# EUROPEAN PATENT SPECIFICATION

(11) **EP 1 844 784 B1**
(45) Date of publication and mention of the grant of the patent: **21.04.2010**
(21) Application number: 06425206.7
(22) Date of filing: 28.03.2006
(51) Int. Cl.: A61K 31/05, A61K 31/164, A61K 31/7032, A61K 9/06, A61K 9/08, A61K 9/14, A61K 9/19, A61K 9/20, A61K 9/48, A61P 37/00, A61P 25/02, A61P 11/00, A61P 17/00, A61P 15/02, A61P 19/02, A61P 29/00

(54) **A pharmaceutical composition for the treatment of pathologies caused by the general response of the immune system**
Eine Pharmazeutische Zusammensetzung zur Behandlung von Pathologien, die durch die allgemeine Immunantwort verursacht werden
Composition pharmaceutique pour le traitement de pathologies causées par une réponse immunitaire

(43) Date of publication of application: 17.10.2007
(73) Proprietor: Epitech Group S.r.l., 20144 Milano (IT)
(72) Inventor: Della Valle, Francesco, 35123 Padova (IT); Della Valle, Maria Federica, 35141 Padova (IT); Marcolongo, Gabriele, 35020 Due Carrare (Padova) (IT); Ravagnan, Gianpiero, 00164 Roma (IT); Di Marzo, Vincenzo, 80078 Pozzuoli (Napoli) (IT)
(74) Representative: Long, Giorgio

(56) References cited:
- WO-A-01/08671
- WO-A-01/28991
- WO-A-95/25736
- US-A- 5 506 224
- US-A- 5 618 842
- US-A1- 2004 014 682
- US-A1- 2005 164 911

## Description

The present invention relates to a pharmaceutical composition comprising amides of mono- and di-carboxylic acids and hydroxystilbenes for the treatment of pathologies caused, sustained and/or characterised by an abnormal general response of the immune system, in both humans and animals.

### STATE OF THE ART

The literature is full of evidence of direct "cross-talk" between the cellular population consisting of mastocytes (M) and the general population of activated T lymphocytes (ATL).

Early evidence for the interaction between M and ATL cells began to emerge at the end of the 90s, from an Israeli immunology research group. In one interesting publication, the authors conclude by hypothesising that the "T cell-mast cell" interaction may be bidirectional and represent an important regulatory and/or modulatory stage of the general immune response (Mekori YA et al. J. Allergy Clin. Immunol. 1999 Sep.;104:517-23). A direct interaction between the two cell lineages was shown for the first time in 2002; mastocyte mediators originating from the degranulation of human M cells are capable of increasing interferon-y production by CD8+ and CD4+ T-lymphocytes (Francina L. de Pater-Huijsen et al. - Immunology 2002 May;106:11-19). This fascinating hypothesis, reiterated in a "review" published in Nature Immunology in February 2005 (Galli S. et al. - Nature Immunology 2005 Feb.;6(2):135-42), is confirmed in the literature, in April 2005 when the same group, belonging to Stephen Galli, show the importance of degranulated TNF from M cells on T lymphocyte activation (Nakae S. et al. - Proc. Natl. Acad. Sci. USA 2005 Apr.;102(18):6467-72); the authors conclude by claiming the identification of a multiple mechanism by means of which mastocytes are capable of influencing the proliferation and production of cytokines from T lymphocytes. The direct "cross-talk" between M cells and T lymphocytes is only clearly confirmed and discussed in October 2005 (Salamon P. et al. - Allergy 2005 Oct.; 60(10):1316-9) and its role in T lymphocyte mediated inflammatory processes demonstrated.

Currently, the diseases characterised by an abnormal immune response are treated by the administration of appropriate drugs which modulate lymphocyte activity exclusively.

The possibility of modulating the general immune response by modulating mastocytes and activated T lymphocytes simultaneously has never been proposed and described.

Thus, the underlying idea behind the present invention is that of modulating the body's general immune response by simultaneously modulating mastocyte (M) reactivity - understood as being the differential degranulatory response of the same - and the activated T lymphocyte (ATL) cytokine and functional response.

Said simultaneous modulation may be obtained through the use of a pharmaceutical composition as described in claim 1.

Hence, the present invention relates to a pharmaceutical composition comprising amides of mono- and di-carboxylic acids and polyphenols belonging to the hydroxystilbene family, and may be used for the treatment of immuno-inflammatory diseases caused, sustained and/or characterised by abnormal general immune responses, in both humans and animals.

In particular, such diseases are those caused by exogenous antigenic stimulii (the so-called "non-self" stimulii). This includes, allergic rhinitis, bronchial asthma, allergic alveolitis, urticaria, atopic dermatitis, contact dermatitis, conjunctivitis and anaphylaxis. Around 10% of the global population suffer from allergic disorders, with an overall predominance in the industrialised countries, with spikes of 15% in subpopulations (infant atopic dermatitis).

Other types of disorders include those caused by endogenous antigenic stimulii (the so-called "self" stimulii), normally defined as autoimmune disorders, characterised by hypersensitivity towards body components. This includes multiple sclerosis, psoriasis, bullous pemphigus, urticaria pigmentosa, systemic scleroderma, uveitis, cicatricial ocular pemphigus, rheumatoid arthritis, systemic lupus erythematosus and psoriatic arthritis. The most frequently observed autoimmune diseases affect no less than 5% of the global population.

The disorders characterised and/or sustained by neuro-immunogenic inflammation - in practice almost all the inflammatory diseases - are organ-specific disorders such as interstitial cystitis in humans and in cats, prostatitis, arthrosis in humans and in dogs, numerous autonomic and somatic neuropathies, vulvovaginitis, vulvar vestibolitis, viral infections of the vagina and uterine neck, oral mucositis, Crohn's disease, ulcerative colitis, geriatric dermatitis (characterised by characteristic symptomatology such as dryness, dyskeratosis, itching, epidermal lesions etc), radiation dermatitis (from the sun, from radiotherapy in cancer patients etc.) and many others. Epidemiological estimates consider the incidence of such diseases to be no less than 40% of the population, and increasing rapidly due to increased average global lifespan and, in the field of veterinary medicine, in consideration of the increasing attention being paid to pets.
The pharmaceutical composition of the invention comprises one or more of the N-acylethanolamine derivatives selected from N-palmitoylethanolamine, N-(2-hydroxyethyl)-lauroylamide, N,N'-bis(2-hydroxyethyl)nonandiamide, N,N'-bis(2-hydroyethyl)-2-dodecendiamide, N,N-bis(2-hydroxyethyl)-lauroylamide and one of more of the hydroxystilbenes selected from resveratrol and the glycosides of resveratrol. The hydroxystilbenes may be in both the *trans* and cis isomeric forms.

The synthesis of the N-acylethanolamine derivatives and hydroxystilbenes of the invention is well known in the art and is, for example, exemplified in EP 0 550 006, in US Patent Application US2004/0014682A1, in US 5,506,224, in US 5,618,842, in EP 0 751 947 and in EP 1 115 392

The one or more N-acylethanolamine derivatives and the one or more hydroxystilbenes are comprised within the composition, respectively, in quantities ranging from 0.0001 mg/kg/day to 20 mg/kg/day, preferably from 0.05 mg/kg/day to 10 mg/kg/day for the N-acylderivatives of aminoalcohols, and preferably from 0.005 mg/kg/day to 10 mg/kg/day for the hydroxystilbenes.

Said active ingredients, optionally micronised or co-micronised with one or more appropriately mixed excipients, may be formulated for oral, buccal, parenteral, rectal, transdermal or topical administration, or in a form adapted for administration by inhalation or insufflation (either through the mouth or nose).

Said appropriately mixed active ingredients may be formulated for oral, buccal, parenteral, rectal, transdermal, topical administration onto the skin and mucosa, or in a form adapted for administration by inhalation or insufflation (either through the mouth or nose).

The pharmaceutical compositions for oral administration may be, for example, in the form of tablets or capsules (containing powders or oil mixtures), acceptable excipients such as binding agents (for example pre-gelatinised corn starch, polyvinylpyrrolidone or carboxymethyl-cellulose); fillers (for example lactose, microcrystalline cellulose or calcium hydrogen phosphate); lubricants (for example magnesium stearate, talc or silica); disintegrants (for example potato starch or sodium starch glycolate); or surfactants (for example sodium lauryl sulphate). Tablets may be coated, using methods well known in the art. Liquid preparations for oral administration may be, for example, in the form of solutions, syrups or suspensions or may be as lyophilised products to be reconstituted, prior to use, with water or other suitable carriers. Such liquid preparations may be obtained using conventional methods with pharmaceutically acceptable additives such as suspension agents (for example sorbitol syrup, cellulose derivatives or hydrogenated edible fats); emulsifiers (for example lecithin or acacia); non-aqueous carriers (for example almond oil, oily esters, ethyl alcohol or fractionated vegetable oils); and preservatives (for example methyl- or propyl-p-hydroxybenzoates or sorbic acid). Preparations may also appropriately contain flavourings, colourants, sweeteners and preservatives.

Preparations for oral administration may be appropriately formulated to allow the controlled release of the active ingredient.

Compositions for buccal administration may be in the form of conventionally formulated tablets or lozenges.

The appropriately mixed active ingredients may be formulated for parenteral administration by injection. Formulations for injections may be presented in single dose form, for example in ampoules with added preservative. The compositions may be presented in the aforementioned form as suspensions, solutions or emulsions in oily or aqueous carriers and may contain formulary agents such as suspension agents, stabilisers and/or dispersants. Alternatively, the active ingredient may be in powder form for reconstitution, prior to use, using an appropriate carrier, for example sterile water.

According to the present invention, the active ingredients may also be formulated in rectal compositions such as suppositories or retention enemas, for example containing common basic suppository components such as cocoa butter or other glycerides.

In addition to the previously described compositions, the compounds may also be formulated as deposit preparations. Such long acting preparations may be administered as implants (for example subcutaneously, transcutaneously or intramuscularly) or by intramuscular injection. Thus, for example, the active ingredients may be formulated with suitable polymeric or hydrophobic materials (for example in the form of an emulsion in a suitable oil) or ion exchange resin or as sparingly soluble derivatives, for example as a sparingly soluble salt.

The appropriately formulated pharmaceutical compositions of the invention may be administered at dosages varying between 0.1 and 30 mg/kg, from 1 to 4 times per day. The dosage of the compositions will be determined according to the disease to be treated, the level of seriousness of said disease, and the condition of the patient. Furthermore, the dose will depend on the selected route of administration. It should be considered that it might be necessary to make continual adjustments to the dosage depending on the age and weight of the patient, in addition to the seriousness of the clinical condition to be treated. The exact dose and the administration route will finally be at the discretion of the physician or veterinarian.

### EXPERIMENTAL SECTION

Some examples of pharmaceutical formulations according to the invention are reported below.

### EXAMPLE 1

### Tablets for oral use.

Each tablet contains:
- co-micronised powder containing:
   - N-palmitoylethanolamine 300 mg
   - Resveratrol 25 mg
   - Resveratrol glucoside 10 mg
   - lactose 200 mg
- corn starch 100 mg
- talc 10 mg
- magnesium stearate 5 mg
- hydroxypropylmethylcellulose 5 mg
- titanium dioxide 1.5 mg
- yellow iron oxide (E172) 0.4 mg

### EXAMPLE 2

### Capsules for oral use.

Each soft gelatine capsule contains:
- N-(2-hydroxyethyl)-lauroylamide 150 mg
- Resveratrol glucoside 50 mg
- soya lecithin 30 mg
- vegetable oil 300 mg
- erythrosine (E127) 0.1 mg

### EXAMPLE 3

### Lyophilised ampoules.

Each lyophilised ampoule contains:
- N,N'-bis-(2-hydroxyethyl)-nonandiamide 100 mg
- Resveratrol glucoside 50 mg
- mannitol 100 mg

Each solvent ampoule contains:
- pyrogen-free double distilled water 3 ml

### EXAMPLE 4

### Ampoules for intramuscular use.

Each ampoule contains:
- N,N'-bis-(2-hydroxyethyl)-nonandiamide 50 mg
- Resveratrol glucoside 50 mg
- physiological solution (isotonic saline) as required up to 2 ml

### EXAMPLE 5

### Cream for dermatological use.

100 g of cream contains:
- N,N'-bis-(2-hydroxyethyl)-nonandiamide 2 g
- Resveratrol 0.5 g
- Resveratrol glucoside 0.3 g
- sorbitan monostearate 0.5 g
- polyoxyethylene-sorbitan-monostearate 4.5 g
- ethyl alcohol 3.0 g
- stearic acid 3.0 g
- paraffin oil 10.0 g
- 70% sorbitol 6.0 g
- methyl p-oxybenzoate 0.2 g
- propyl p-oxybenzoate 0.05 g
- deionised water as required up to 100.0 g

### EXAMPLE 6

### Gel for gynaecological use.

100 g of gel contains:
- N,N'-bis-(2-hydroxyethyl)-nonandiamide 1.0 g
- Resveratrol glucoside 1.0 g
- glycerine 8.0 g
- Hydrogenated ricin oil 1.0 g
- Propylene glycol 1.0 g
- polycarbophil 1.5 g
- vitamin E acetate 0.5 g
- Vitamin A acetate 0.05 g
- polyvinyl alcohol 0.2 g
- hyaluronic acid 0.2 g
- phytosphingosine 0.02 g
- methyl p-oxybenzoate 0.10 g
- 2-phenylethanol 0.15 g
- quercetin 0.1 g
- water as required up to 100.0 g

### EXAMPLE 7

### Soft gelatin vaginal ovules.

Each ovule contains:
- N,N'-bis-(2-hydroxyethyl)-trans-2-dodecenediamide 0.5 g
- resveratrol glucoside 0.6 g
- propylene glycol 1.5 g
- erythrosine (E127) 0.1 mg

### EXAMPLE 8

### Ophthalmic ointment.

100 g of ointment contains:
- N,N'-bis-(2-hydroxyethyl)-trans-2-dodecenediamide 0.5 g
- Resveratrol 0.5 g
- viscous vaseline as required up to 100.0 g

### EXAMPLE 9

### Sterile ophthalmic eyewash (in single dose packs).

100 g of eyewash contains:
- N,N'-bis-(2-hydroxyethyl)-nonandiamide 0.5 g
- resveratrol glucoside 0.3 g
- hyaluronic acid sodium salt 0.01 g
- pyrogen-free physiological solution as required up to 100.0 g

### EXAMPLE 10

### Rectal suppositories.

Each suppository contains:
- N-palmitoylethanolamine 0.3 mg
- resveratrol 0.2 mg
- resveratrol glucoside 0.05 mg
- Fatty excipient for suppositories
- as required up to 2.0 g

### EXAMPLE 11

### Powder for chiropody use.

100 g of powder contains:
- co-micronised powder containing:
   - N,N'-bis-(2-hydroxyethyl)-lauroylamide 2.3 g
   - resveratrol 1.5 g
   - resveratrol glucoside 0.8 g
   - attapulgite 50.0 g
- acrylic copolymer 3.0 g
- vitamin E acetate 1.0 g
- dimethicone 2.0 g
- isopropylmyristate 2.0 g
- menthol 1.5 g
- eucalyptol 0.5 g
- micronised attapulgite as required up to 100.0 g

### EXAMPLE 12

### Nail drop solution

100 g of solution contains:
- N,N'-bis-(2-hydroxyethyl)-nonandiamide 2.0 g
- N,N'-bis-(2-hydroxyethyl)-lauroylamide 1.0 g
- resveratrol glucoside 2.0 g
- phytosphingosine 1.0 g
- isopropyl alcohol 80.0 g
- acetone 2.0 g
- undecylenic acid 0.2 g
- vitamin A palmitate 0.5 g
- urea 3.0 g
- deionised water as required up to 100.0 g

## Claims

1. A pharmaceutical composition comprising one or more of the N-acylethanolamine derivatives selected from N-palmitoylethanolamine, N-(2-hydroxyethyl)-lauroylamide, N,N'-bis(2-hydroxyethyl)nonandiamide, N,N'-bis(2-hydroxyethyl)-2-dodecendiamide, N,N-bis(2-hydroxyethyl)-lauroylamide and one or more of the hydroxystilbenes selected from resveratrol and the glycosides of resveratrol.

2. The composition according to claim 1 wherein the resveratrol or glycosides of resveratrol are *trans* isomers and *cis* isomers.

3. The composition according to claim 1 or 2, wherein said one or more N-acylethanolamine are included in quantities ranging from 0.0001 mg/Kg/day to 20 mg/Kg/day, preferably from 0.05 mg/Kg/day to 10 mg/Kg/day, and said one or more hydroxystilbenes are included in quantities ranging from 0.005 mg/Kg/day to 10 mg/Kg/day.

4. A pharmaceutical formulation comprising the composition according to claims 1 to 3 together with pharmaceutically acceptable excipients.

5. The formulation according to claim 4 for oral, buccal, parenteral, rectal, transdermal, topical application onto skin and mucosa, for inhalation or insufflation.

6. The composition according to claims 1 to 3 for use as a medicament.

7. Use of the composition according to claims 1 to 3 for the preparation of a medicament for the treatment of diseases caused, sustained and/or **characterised by** the abnormal general response of the immune system in humans and animals, wherein said diseases are caused by exogenous antigenic stimulii, preferably: allergic rhinitis, bronchial asthma, allergic alveolitis, urticaria, atopic dermatitis, contact dermatitis, conjunctivitis, anaphylaxis; diseases caused by endogenous antigenic stimulii (autoimmune diseases), preferably multiple sclerosis, psoriasis, bullous pemphigus, urticaria pigmentosa, systemic scleroderma, uveitis, cicatricial ocular pemphigus, rheumatoid arthritis, systemic lupus erythematosus, psoriatic arthritis; diseases **characterised** and/or sustained by neuro-immunogenic inflammation, preferably interstitial cystitis in humans and in cats, prostatitis, arthrosis in humans and in dogs, numerous autonomic and somatic neuropathies, vulvovaginitis, viral infections of the vagina and uterine neck, vulvar vestibolitis, oral mucositis, Crohn's disease, ulcerative colitis, geriatric dermatitis, solar radiation or radiotherapy induced dermatitis.

## Patentansprüche

1. Pharmazeutische Zusammensetzung, umfassend eines oder mehrere der N-Acylethanolaminderivate, die aus N-Palmitoylethanolamin, N-(2-Hydroxyethyl)-lauroylamid, N,N'-bis(2-Hydroxyethyl)-nonandiamid, N,N'-bis(2-Hydroxyethyl)-2-dodecendiamid, N,N-bis(2-Hydroxyethyl)-lauroylamid ausgewählt sind, und eines oder mehrere der Hydroxystilbene, die aus Resveratrol und den Glycosiden von Resveratrol ausgewählt sind.

2. Zusammensetzung nach Anspruch 1, worin das Resveratrol oder die Glycoside von Resveratrol *trans*-Isomere und *cis*-Isomere sind.

3. Zusammensetzung nach Anspruch 1 oder 2, worin das eine oder die mehreren N-Acylethanolamin(e) in Mengen im Bereich von 0,0001 mg/kg/Tag bis 20 mg/kg/Tag, bevorzugt von 0,05 mg/kg/Tag bis 10 mg/kg/Tag enthalten ist/sind, und das eine oder die mehreren Hydroxystilben(e) in Mengen im Bereich von 0,005 mg/kg/Tag bis 10 mg/kg/Tag enthalten ist/sind.

4. Pharmazeutische Formulierung, umfassend die Zusammensetzung nach einem der Ansprüche 1 bis 3, zusammen mit pharmazeutisch akzeptablen Hilfsstoffen.

5. Formulierung nach Anspruch 4 zur oralen, buccalen, parenteralen, rektalen, transdermalen, topischen Anwendung auf der Haut und Schleimhaut, zur Inhalation oder Insufflation.

6. Zusammensetzung nach den Ansprüchen 1 bis 3 zur Verwendung als Medikament.

7. Verwendung der Zusammensetzung nach den Ansprüchen 1 bis 3 zur Herstellung eines Medikaments zur Behandlung von Krankheiten, die durch die abnormale allgemeine Antwort des Immunsystems in Menschen und Tieren verursacht werden, aufrechterhalten werden und/oder **gekennzeichnet** sind, wobei die Krankheiten durch exogene antigene Stimuli verursacht werden, bevorzugt: allergische Rhinitis, Bronchialasthma, allergische Alveolitis, Urticaria, atopische Dermatitis, Kontaktdermatitis, Konjunktivitis, Anaphylaxie; wobei die Krankheiten, durch endogene antigene Stimuli verursacht werden (Autoimmunkrankheiten), bevorzugt Multiple Sklerose, Psoriasis, blasiger Pemphigus, Urticaria pigmentosa, systemische Sklerodermie, Uveitis, vernarbender okulärer Pemphigus, rheumatoide Arthritis, systemischer Lupus erythematodes, psoriatische Arthritis; wobei die Krankheiten durch neuroimmunogene Entzündung **gekennzeichnet** sind und/oder aufrechterhalten werden, bevorzugt Interstitielle Cystitis in Menschen und in Katzen, Prostatitis, Arthrose in Menschen und in Hunden, zahlreiche autonome und somatische Neuropathien, Vulvovaginitis, Virusinfektionen der Vagina und des Gebärmutterhalses, Vulvar Vestibulitis, orale Mucositis, Morbus Crohn, Colitis ulcerosa, geriatrische Dermatitis, durch Sonneneinstrahlung oder Strahlentherapie induzierte Dermatitis.

## Revendications

1. Composition pharmaceutique comprenant un ou plusieurs des dérivés de la N-acyléthanolamine sélectionnés parmi la N-palmitoyléthanolamine, le N-(2-hydroxyéthyl)-lauroylamide, le N,N'-bis(2-hydroxyéthyl)nonanediamide, le N,N'-bis(2-hydroxyéthyl)-2-dodécènediamide, le N,N'-bis(2-hydroxyéthyl)-lauroylamide et un ou plusieurs des hydroxystilbènes sélectionnés parmi le resvératrol et les glycosides du resvératrol.

2. Composition selon la revendication 1 où le resvératrol ou les glycosides du resvératrol sont des isomères *trans* et des isomères *cis*.

3. Composition selon la revendication 1 ou 2, où lesdites une ou plusieurs N-acyléthanolamines sont incluses en des quantités allant de 0,0001 mg/kg/jour à 20 mg/kg/jour, de préférence de 0,05 mg/kg/jour à 10 mg/kg/jour, et lesdits un ou plusieurs hydroxystilbènes sont inclus en des quantités allant de 0,005 mg/kg/jour à 10 mg/kg/jour.

4. Formulation pharmaceutique comprenant la composition selon les revendications 1 à 3 avec des excipients pharmaceutiquement acceptables.

5. Formulation selon la revendication 4 pour application orale, buccale, parentérale, rectale, transdermique, topique sur la peau et les muqueuses, pour inhalation ou insufflation.

6. Composition selon les revendications 1 à 3 pour utilisation comme médicament.

7. Utilisation de la composition selon les revendications 1 à 3 pour la préparation d'un médicament destiné au traitement de pathologies provoquées, entretenues et/ou **caractérisées par** une réaction générale anormale du système immunitaire chez l'humain et l'animal, où lesdites pathologies sont des pathologies provoquées par des stimulus antigéniques exogènes, de préférence: rhinite allergique, asthme bronchique, alvéolite allergique, urticaire, eczéma atopique, eczéma de contact, conjonctivite, anaphylaxie; de pathologies provoquées par des stimulus antigéniques endogènes (maladies auto-immunes), de préférence sclérose en plaques, psoriasis, pemphigus bulleux, urticaire pigmentaire, sclérodermie systémique, uvéite, pemphigus oculaire cicatriciel, polyarthrite rhumatoïde, lupus érythémateux systémique, rhumatisme psoriasique ; des pathologies **caractérisées** et/ou entretenues par une inflammation neuro-immunogène, de préférence cystite interstitielle chez l'humain et le chat, prostatite, arthrose chez l'humain et le chien, nombreuses névropathies autonomes et somatiques, vulvovaginite, infections virales du vagin et du col de l'utérus, vestibulite vulvaire, inflammation de la muqueuse orale, maladie de Crohn, rectocolite hémorragique, eczéma des personnes âgées, actinite solaire ou radiodermite.
